# EUROPEAN PATENT APPLICATION

(11) **EP 2 224 017 A1**
(43) Date of publication of application: **01.09.2010**
(21) Application number: 08865229.2
(22) Date of filing: 25.12.2008
(51) Int. Cl.: C12Q 1/68, C12N 15/09

(54) **METHOD FOR AMPLIFYING TARGET NUCLEIC ACID SEQUENCE AND PROBE USED FOR THE SAME**

(30) Priority: 26.12.2007 JP 2007334360
(71) Applicant: ARKRAY, Inc., Kyoto-shi, Kyoto 601-8045 (JP)
(72) Inventor: HIRAI Mitsuharu, Kyoto-shi, Kyoto 601-8045 (JP); MAJIMA Satoshi, Kyoto-shi, Kyoto 601-8045 (JP); HOSOMI Toshiya, Kyoto-shi, Kyoto 601-8045 (JP)
(74) Representative: Jones, Elizabeth Louise
(86) International application number: PCT/JP2008/073537
(87) International publication number: WO 2009/081967

(57) **Abstract**

The present invention provides a method for amplifying a target sequence while suppressing inhibition of an amplification reaction in nucleic acid amplification in the presence of the probe. At the time of amplifying the target sequence, as the probe caused to coexist in amplification of the target sequence, a probe having a base sequence in which a melting temperature of the double-stranded nucleic acid is equal to or lower than a reaction temperature of the elongation reaction is used. In the presence of such a probe, for example, annealing of a primer and an elongation reaction from the primer are hardly inhibited by the presence of the probe so that amplification of the target sequence can be conducted sufficiently. Therefore, when a polymorphism of a target site in the target sequence is analyzed by a Tm analysis or the like, high reliability can be achieved.

## Description

### Technical Field

The present invention relates to a method for amplifying a target nucleic acid sequence in the presence of a probe and a probe used therefor. In addition, the present invention relates to a method for suppressing the inhibition of amplification of a target nucleic acid sequence in the presence of a probe and a method for analyzing a target nucleic acid sequence.

### Background Art

In recent years, as a method for detecting a mutation or a polymorphism in a gene, a method for analyzing a melting temperature (Tm) of a double-stranded nucleic acid composed of a target nucleic acid sequence and a detection probe has been put to practical use. The foregoing method is, for example, referred to as a melting profile analysis or a Tm analysis because the method is conducted by analyzing a melting profile of the double strand. A Tm generally is defined as follows. The absorbance at 260 nm increases as a solution containing a double-stranded nucleic acid such as a double-stranded DNA or the like is heated. This increase is caused by the fact that the hydrogen bond between both the strands in a double-stranded nucleic acid is unbound by heating, and the double-stranded nucleic acid is dissociated into single-stranded nucleic acids (melting of a nucleic acid). When every double-stranded nucleic acid is dissociated into single-stranded nucleic acids, the solution exhibits an absorbance about 1.5 times as large as the absorbance at the time when the heating was initiated. (the absorbance of the solution containing only the double-stranded nucleic acid), whereby it can be determined that the melting is completed. Based on this phenomenon, a melting temperature, (Tm) (°C), generally is defined as a temperature at the time when the amount of increase in absorbance reaches 50% of the total amount of increase in absorbance.

A single nucleotide polymorphism occurs by substitution of a single base (point mutation) in a site to be detected (hereinafter simply referred to as "target site"). Therefore, in order to analyze the type of the polymorphism in the target site, it is necessary to distinguish the difference in a single base. In the Tm analysis described above, this difference in a single base is analyzed by the difference in Tm value. It is described specifically below. A polymorphism in a target site is referred to as a mutant type or a wild type for the sake of convenience.

The Tm value becomes higher as the homology of a double-stranded nucleic acid becomes higher and becomes lower as the homology of a double-stranded nucleic acid becomes lower. Therefore, for example, in the case where a detection probe that is perfectly complementary to a target nucleic acid sequence having a mutant-type target site (hereinafter referred to as "mutant-type probe") is designed, the Tmₘᵤₜ value of a double-stranded nucleic acid composed of the mutant-type probe and the target sequence with a mutant-type target site is higher than the Tm_{wild} value of a double-stranded nucleic acid composed of the mutant-type probe and a target sequence with a wild-type target site. According to the Tm analysis utilizing this nature, a polymorphism can be analyzed in the following manner. First, a Tmₘᵤₜ value and a Tm_{wild} value in the case where the mutant-type probe is used are previously determined as evaluation criteria. Subsequently, a double-stranded nucleic acid composed of a target nucleic acid sequence to be analyzed having an unknown base in a target site and the mutant-type probe is formed, a heat treatment is conducted to the formed double-stranded nucleic acid, and signal values such as absorbance and the like showing disassociation of the double-stranded nucleic acid with increase in temperature are measured. Further, a melting profile showing variations in signal value with changes in temperature is prepared, and whether a peak is present at the previously determined Tmₘᵤₜ value or at the previously determined Tm_{wild} value is checked. When the peak is present at around the Tmₘᵤₜ value, the target nucleic acid sequence is a 100% match to the mutant-type probe, whereby the target site in the target nucleic acid sequence can be determined as having a mutant-type polymorphism. On the other hand, when the peak is present at around the Tm_{wild} value, the target nucleic acid sequence is a mismatch to the mutant-type probe in a single base, whereby the target site in the target nucleic acid sequence can be determined as having a wild-type polymorphism. Further, whether the polymorphism is homozygous or heterozygous also can be determined. That is, in an analysis of a polymorphism in a pair of alleles, when peaks are present at both around the Tmₘᵤₜ value and around the Tm_{wild} value, the polymorphism can be determined as being heterozygous. On the other hand, when a peak is present at only around the Tmₘᵤₜ value, the polymorphism can be determined as being a mutant-type homozygous, and when a peak is present at only around the Tm_{wild} value, the polymorphism can be determined as being a wild-type homozygous. Further, also in the case where a detection probe that is perfectly complementary to a target nucleic acid sequence having a wild-type target site (hereinafter referred to as "wild-type probe") is used, by setting, as evaluation criteria, the Tm_{wild} value of a double-stranded nucleic acid composed of the wild-type probe and a target sequence with a wild-type target site and the Tmₘᵤₜ value of a double-stranded nucleic acid composed of the wild-type probe and the target sequence with a mutant-type target site, the determination can be made in the same manner. It is to be noted that, in the case of the wild-type probe, the Tm_{wild} value of a double-stranded nucleic acid composed of the wild-type probe and the target sequence with a wild-type target site is higher than the Tmₘᵤₜ value of a double-stranded nucleic acid composed of the wild-type probe and the target sequence with a mutant-type target site.

In a method for analyzing a polymorphism utilizing such a Tm analysis, in order to further simplify the analysis, an amplification reaction of the target nucleic acid sequence is conducted in the presence of the detection probe, and signal values are measured using the resultant reaction solution after the amplification reaction as it is. According to such a method, a simplified analysis can be achieved because, after the amplification reaction, a step of further adding the detection probe into the resultant reaction solution can be omitted. Further, when the detection probe is previously added in the reaction solution, airtight state of the reaction solution can be maintained, for example, whereby prevention of contamination can be achieved.
Patent Citation 1: JP 2005-58107 A

### Disclosure of Invention

However, comparing a method in which an amplification reaction is conducted in the absence of a probe and a Tm analysis is conducted by adding the probe to the resultant reaction solution and a method in which an amplification reaction is conducted in the presence of a probe and a Tm analysis is conducted using the resultant reaction solution, it was found that, for example, even if the same template nucleic acid and probe are used, the following problem may arise in the latter method. Specifically, the problem is that, even in the case where a target nucleic acid sequence with a known polymorphism is used, i.e., it is known whether the peak is found at either the Tm_{wild} value or the Tmₘᵤₜ value, it is difficult to distinguish the peak because it is too small, or the peak is not found. Especially, in the case of a sample known to be heterozygous, it is theoretically considered that peaks comparable to each other are seen at both the Tm_{wild} value and the Tmₘᵤₜ value. However, in fact, there is a case where a peak of a target sequence perfectly complementary to the probe (for example, in the case where the mutant-type probe is used, it is a peak at the Tmₘᵤₜ value) is hardly distinguishable.

Hence, the present invention is intended to provide an amplification method that can amplify a target nucleic acid sequence even in the presence of a probe, a method for suppressing amplification inhibition caused by the probe, a method for analyzing a target nucleic acid sequence, and the probe used therefor.

In order to achieve the aforementioned object, the amplification method of the present invention is a method for amplifying a target nucleic acid sequence in a template nucleic acid in the presence of a probe, including the step of: amplifying the target nucleic acid sequence by an elongation reaction from a primer in the presence of a probe that can hybridize to the target nucleic acid sequence. In this method, used as the probe is a probe that forms, with a strand complementary to the probe, a double-stranded nucleic acid whose melting temperature is equal to or lower than a reaction temperature of the elongation reaction. In the present invention, hereinafter, a target nucleic acid sequence also is referred to as a target sequence.

The method for suppressing amplification inhibition of the present invention is a method for suppressing, in amplification of a target nucleic acid sequence in a template nucleic acid in the presence of a probe, inhibition of the amplification of the target nucleic acid sequence, wherein a method for amplifying the target nucleic acid sequence is the amplification method of the present invention.

The analysis method of the present invention is a method for analyzing a target nucleic acid sequence using a probe that can hybridize to the target nucleic acid sequence, including the steps of: (A) amplifying the target nucleic acid sequence in a template nucleic acid in the presence of the probe by the amplification method according to the present invention; and (B) after the step (A), measuring a signal value showing a molten state of a double-stranded nucleic acid composed of a resultant amplification product and the probe while changing a temperature of a reaction solution in the step (A).

The probe of the present invention is a probe used for the amplification method of the present invention. The probe is a probe that forms, with a strand complementary to the probe, a double-stranded nucleic acid whose melting temperature is equal to or lower than a reaction temperature of the elongation reaction.

The inventors of the present invention conducted earnest studies to elucidate the cause of the above-described problem in the case where an amplification reaction is conducted in the presence of the probe and a Tm analysis is conducted using the resultant reaction solution. As a result of the studies, they found a possibility that a probe previously added to the reaction solution before the amplification reaction hybridizes to a template nucleic acid at the time of the amplification reaction, thereby inhibiting, for example, annealing of a primer or elongation from the annealed primer. It is considered that, if annealing of a primer to the template nucleic acid or elongation from the primer is inhibited, even when the target sequence is present, the amplification thereof does not occur sufficiently, thereby causing a problem of a peak being difficult to detect. Based on this finding, the inventors found that the problem can be avoided by designing a probe so that the Tm value thereof and the elongation reaction temperature satisfy the relationship described above. This is for the following reasons.

In a polymorphism analysis using a probe, in order to enhance the accuracy of the analysis, a probe is usually designed so as to hybridize specifically to a target sequence. In order to allow a probe to hybridize specifically to a target sequence, a generally employed approach is to make a probe relatively long. However, as the probe becomes relatively long, the Tm value thereof (i.e., for example, the Tm value of a double-stranded nucleic acid composed of the probe and a strand perfectly complementary to the probe) becomes relatively high, so that, once a double strand is formed, the probe is difficult to dissociate from a template unless a higher temperature is applied thereto. The inventors found that the conventional problem of difficulty in detecting a peak is caused by the fact that a probe that is designed so as to specifically hybridize to the target nucleic acid for a Tm analysis of a polymorphism after nucleic acid amplification hybridizes to the template nucleic acid at the time of nucleic acid amplification, thereby inhibiting annealing of a primer and an elongation reaction from the annealed primer. Thus, in order to make a probe less prone to hybridize to a template nucleic acid at least in an elongation reaction in nucleic acid amplification, the base sequence of the probe is set so that the Tm value thereof, i.e., the Tm value of a double-stranded nucleic acid composed of the probe and a strand complementary to the probe (for example, a strand perfectly complementary to the probe), is equal to or lower than the elongation reaction temperature, thereby achieving the present invention. According to the present invention, even if amplification of a target sequence is conducted in the presence of a probe, the probe is less prone to hybridize to a template nucleic acid at a reaction temperature of the elongation reaction. Therefore, nucleic acid amplification of a target sequence hardly is inhibited by the probe, specifically, for example, annealing of a primer to a template nucleic acid and an elongation reaction from the primer are hardly inhibited. As a result, it becomes possible to amplify the target sequence sufficiently. In particular, comparing the Tm value of a double-stranded nucleic acid composed of the probe and a strand perfectly complementary to the probe and the Tm value of a double-stranded nucleic acid composed of the probe and a strand complementary to the probe except for a single base, the former Tm value is higher than the latter Tm value. Thus, conventionally, it is considered that a target sequence perfectly complementary to a probe is more susceptible to influence by the probe than a target sequence complementary to the probe except for a single base. However, according to the probe of the present invention, such a problem is avoided and hence, for example, even in the case of a heterozygous template nucleic acid as described above, it becomes possible to achieve the same amplification efficiency and also, it becomes possible to detect peaks in a Tm analysis. Therefore, according to the present invention, for example, in a Tm analysis, the presence or absence of a peak can be distinguished with higher reliability than ever before. Consequently, it can be said that the present invention is very useful technology especially in the field of gene analysis.

The problem caused in the Tm analysis in the case where a nucleic acid amplification is conducted in the presence of a probe and the cause of the problem are newly found by the inventers. Further, although there has been a probe designed so that the Tm value is relatively high in order to allow the probe to specifically hybridize to a target sequence, an attempt to design a probe considering the temperature relation between the Tm value and the reaction temperature of the elongation reaction in order to avoid the influence of the probe on nucleic acid amplification is made first in the present invention.

### Brief Description of Drawings

[FIG. 1] FIG. 1 is a graph showing a result of a Tm analysis in a comparative example.
[FIG. 2] FIG. 2 is a graph showing a result of a Tm analysis in an example of the present invention.
[FIG. 3] FIG. 3 is a graph showing another result of a Tm analysis in an example of the present invention.

### Best Mode for Carrying out the Invention

### <Amplification method>

The amplification method of the present invention is, as described above, a method for amplifying a target nucleic acid sequence in a template nucleic acid in the presence of a probe, including the step of: amplifying the target nucleic acid sequence by an elongation reaction from a primer in the presence of the probe described above. The probe used in this method is the probe of the present invention, i.e., a probe that forms, with a strand complementary to the probe, a double-stranded nucleic acid whose melting temperature is equal to or lower than a reaction temperature of the elongation reaction. The probe used in the present invention is, hereinafter, referred to as a probe of the present invention. In an amplification method of the present invention, the probe preferably is a probe that forms, with a strand perfectly complementary to the probe, a double-stranded nucleic acid whose melting temperature is equal to or lower than a reaction temperature of the elongation reaction.

By conducting a nucleic acid amplification reaction in the presence of the probe of the present invention, as described above, for example, amplification of a target sequence can be conducted while suppressing annealing of a primer to a template nucleic acid and inhibition of elongation from the annealed primer. It is to be noted that the amplification method of the present invention is characterized by using a probe that satisfies the condition described above, and types, conditions, and the like of the nucleic acid amplification method are not limited at all.

In the present invention, in order to identify a probe to be used, the probe is defined as having a property such that, when a double-stranded nucleic acid composed of the probe and a strand perfectly complementary to the probe is formed, "a melting temperature of the double-stranded nucleic acid composed of the probe and the strand perfectly complementary to the probe is equal to or lower than a reaction temperature of the elongation reaction". That is, the "perfectly complementary strand" merely is a condition to identify the property of a probe to be used, and it is not required that the probe forms a double-stranded nucleic acid with the perfectly complementary strand at the time of use. As described above, for example, it is not necessary that the present invention includes a step of forming a double-stranded nucleic acid from the probe and a strand perfectly complementary to the probe. In addition, for example, in a method for analyzing a target nucleic acid sequence to be described later and the like, when a double strand composed of an amplification product after an amplification reaction and the probe is formed, an object to which the probe hybridizes is not limited to the strand perfectly complementary to the probe, and the probe may hybridize to a complementary strand with a single-base mismatch or multiple-base mismatches.

In the present invention, hereinafter, the state of being perfectly complementary to the probe is referred to as "perfect match", a complementary strand perfectly matching to the probe is referred to as "perfectly matched complementary strand", a double-stranded nucleic acid composed of the probe and the perfectly matched complementary strand is referred to as "double-stranded nucleic acid of perfect match", and a Tm value of the double-stranded nucleic acid of perfect match is referred to as "Tm_{Per} value". The state of being perfectly complementary to the probe except for a single base or multiple bases is referred to as "mismatch", a complementary strand mismatching the probe is referred to as "mismatched complementary strand", a double-stranded nucleic acid composed of the probe and the mismatched complementary strand is referred to as "double-stranded nucleic acid of mismatch", and a Tm value of the double-stranded nucleic acid of mismatch is referred to as "Tm_{Mis} value". In addition, in the present invention, a sequence amplified by a nucleic acid amplification method may be a target sequence and a sequence complementary thereto, or may be a sequence composed of a region including the target sequence and a sequence complementary thereto.

The probe of the present invention may be a probe designed such that, for example, the relation between the Tm_{Per} value of the double-stranded nucleic acid of perfect match and the elongation reaction temperature satisfies the condition "Tm_{Per} value ≤ elongation reaction temperature (°C)", and a base sequence of the probe or the number of bases in the same is not limited at all. Nucleic acid amplification generally includes three steps, namely, melting of a double-stranded nucleic acid (disassociation to single-stranded nucleic acids), annealing of primers to the single-stranded nucleic acids, and an elongation reaction of complementary strands from the annealed primers. The annealing is a precondition of the elongation reaction, and in the nucleic acid amplification, generally, the temperature of annealing and the reaction temperature of an elongation reaction are set to be the same. In this case, "elongation reaction temperature" in the present invention can be replaced with the annealing temperature, for example.

For example, when the reaction temperature of an elongation reaction has been set to a particular value, the base sequence of the probe of the present invention can be designed so that the Tm value is equal to or lower than the elongation reaction temperature. The method for designing a probe taking the Tm value into account is not particularly limited, and a conventionally known method can be adopted. The sequence of the probe or the Tm value can be determined by the conventionally known MELTCALC software (http://www.meltcalc.com/), the conventionally known Nearest Neighbor Method, or the like. With respect to the probe designed using the foregoing software or the like, the Tm_{Per} value is preferably checked, for example, under the actual condition of conducting nucleic acid amplification. Further, the probe of the present invention may be adapted to satisfy the condition of the present invention by setting the elongation reaction temperature depending on the Tm_{Per} value of the probe, instead of determining the base sequence depending on the elongation reaction temperature as described above. That is, with respect to the probe of the present invention, it is only necessary that the Tm_{Per} value of the probe and the elongation reaction temperature eventually satisfy the formula "Tm_{Per} value ≤ elongation reaction temperature", and the means for allowing the formula to be satisfied may be either adjusting the base sequence of the probe or adjusting the elongation reaction temperature.

The Tm_{Per} value of a double-stranded nucleic acid composed of the probe and the perfectly matched complementary strand may satisfy the condition "Tm_{Per} value ≤ elongation reaction temperature". The difference between the Tm_{Per} value and the elongation reaction temperature is, for example, 0°C or higher (about 0°C or higher). The upper limit of the difference is not particularly limited, and is, for example, preferably from 0°C to 30°C, more preferably from 0°C to 20°C, still more preferably from 0°C to 10°C, and particularly preferably from 0°C to 2°C.

Since the homology of the mismatched complementary strand to the probe is lower than that of the perfectly matched complementary strand, the Tm_{Mis} value is lower than the Tm_{Per} value (Tm_{Mis} value < Tm_{Per} value < elongation reaction temperature (°C)). With respect to the probe of the present invention, the difference between the Tm_{Per} value of a double-stranded nucleic acid composed of the probe and the perfectly matched complementary strand and the Tm_{Mis} value of a double-stranded nucleic acid composed of the probe and the mismatched complementary strand is, for example, preferably 1°C or higher (about 1°C or higher), more preferably 3°C or higher (about 3°C or higher), and particularly preferably 5°C or higher (about 5°C or higher). By providing enough difference between the Tm_{Per} value and the Tm_{Mis} value, for example, when a melting profile to be described later is prepared, a peak of the Tm_{Per} value and a peak of the Tm_{Mis} value can be determined easily, whereby the accuracy of analysis is enhanced further.

As long as the Tm_{Per} value of the probe and the elongation reaction temperature satisfy the relation described above, the length of the probe is not particularly limited. It is preferable that the sequence of the probe is relatively long because the probe can hybridize to a target sequence more specifically. The length is, for example, from 5 to 50 bases, and preferably from 10 to 30 bases.

The probe of the present invention may be, for example, a non-labeled probe, or a labeled probe labeled with a labeling substance, and specifically, the labeled probe is preferred. According to such a labeled probe, for example, in the method for analyzing a target sequence to be described later, signals of the labeling substance can be measured as signal values showing molten states of a double-stranded nucleic acid composed of the amplification product and the probe.

Examples of the labeling substance include fluorescent substances such as a fluorescence dye, a fluorophore, and the like. As a specific example of the labeled probe, for example, a probe labeled with the fluorescent substance, showing fluorescence independently and exhibiting reduced (for example, quenched) fluorescence by hybrid formation is preferred. A probe utilizing such a quenching phenomenon is generally called a fluorescence quenching probe. Among them, preferred is the probe labeled with the fluorescent substance at the 3' terminal of a polynucleotide or the 5'terminal of the same, and the terminal base that is labeled is preferred to be C. In this case, preferably, the base sequence of the labeled probe is designed so that, in a base sequence to which the labeled probe hybridizes, a base to be paired with the terminal base C of the labeled probe or a base that is 1 base to 3 bases away therefrom is G. The foregoing probe is generally called a guanine quenching probe, and it is known as the so-called QProbe (registered trademark). When such a guanine quenching probe hybridizes to a base sequence, the phenomenon that light emission of the fluorescent substance becomes weak (the fluorescence intensity is reduced) as the terminal C labeled with a fluorescent substance approaches the G in the base sequence is occurs. By using such a probe, hybridization and disassociation can be recognized easily by variations in signal.

The fluorescent substance is not particularly limited, and examples thereof include fluorescent dyes such as fluorescein, phosphor, rhodamine, polymethine dye derivatives, and the like. Examples of a commercially available fluorescent substance include fluorescent dyes such as BODYPY FL (trademark, produced by Molecular Probes, Inc.), FluorePrime (product name, produced by Amersham Pharmacia Biotech, Inc.), Fluoredite (product name, produced by Millipore Corporation), FAM (produced by ABI Inc.), Cy 3 and Cy 5 (produced by Amersham pharmacia), TAMRA (produced by Molecular Probes, Inc.), and the like.

When two or more types of the probes of the present invention are used in one reaction system, for example, the probes are preferably labeled with different fluorescent substances from each other (fluorescent substances detected at different wavelengths from each other). The combination of the fluorescent substances is not particularly limited as long as, for example, the fluorescent substances can be detected under different conditions from each other. Examples thereof include the combination of Pacific Blue (the detection wavelength is from 450 to 480 nm), TAMRA (the detection wavelength is from 585 to 700 nm), and BODIPY FL (the detection wavelength is from 515 to 555 nm), and the like.

Further, in a labeled probe labeled with a fluorescent dye at the 5' terminal, for example, a phosphate group further may be added to the 3' terminal to prevent elongation of the probe itself in an amplification reaction.

In the present invention, the nucleic acid amplification method is not particularly limited, and examples thereof include a polymerase chain reaction (PCR) method, a nucleic acid sequence based amplification (NASBA) method, a transcription-mediated amplification (TMA) method, a strand displacement amplification (SDA) method, and the like. Among them, the PCR method is preferred. Hereinafter, the present invention will be explained with the PCR method as an example. However, the present invention is not limited to this example.

A sample to which the present invention is applied is not particularly limited as long as it contains a nucleic acid serving as a template. Specific examples thereof include: whole blood, somatic cells such as oral cells (for example, oral mucosa), nails, hair, and the like; germ cells; sputa; amniotic fluid; paraffin-embedded tissues, urine, gastric juice (for example, gastric lavage fluid), and the like, suspensions thereof, and the like.

The proportion of the sample added in a reaction solution for nucleic acid amplification is not particularly limited. As a specific example, when the sample is a biological sample (for example, whole blood sample), the lower limit of the proportion thereof in the reaction solution is preferably 0.01% by volume or more, more preferably 0.05% by volume or more, and still more preferably 0.1% by volume or more. Further, the upper limit of the proportion thereof also is not particularly limited, and is, for example, preferably 2% by volume or less, more preferably 1% by volume or less, and still more preferably 0.5% by volume or less.

Further, as will be described later, when optical detection is conducted with respect to a reaction solution of nucleic acid amplification, especially when an optical detection using a labeled probe is conducted, preferably, the proportion of a biological sample such as a whole blood sample or the like added in the reaction solution is, for example, set to from 0.1% to 0.5% by volume. In a PCR, usually a heat treatment is conducted to denature a nucleic acid (disassociation to single-stranded nucleic acids). However, there is a risk that, by this heat treatment, sugars, proteins, and the like contained in the sample are denatured, resulting in the generation of insoluble deposits, turbidity, or the like. Therefore, when the presence or absence of an amplification product or a polymorphism of the same is checked by an optical method, the generation of insoluble deposits or turbidity as described above may influence the accuracy of measurement. However, when the proportion of a whole blood sample added in a reaction solution is set in the range described above, for example, the influence of generated insoluble deposits or the like caused by the denaturation can be prevented sufficiently, although the mechanism thereof is unknown, whereby the accuracy of the measurement by an optical method can be enhanced. Further, since the inhibition of a PCR by foreign substances in the whole blood sample also is suppressed sufficiently, amplification efficiency can be enhanced further.

Further, the proportion of a whole blood sample in the reaction solution also can be expressed as percentage by weight of hemoglobin (hereinafter referred to as "Hb"), instead of percentage by volume as described above (for example, from 0.1% to 0.5% by volume). In this case, the proportion of the whole blood sample in the reaction solution is converted to the amount of Hb, which is, for example, preferably in a range of from 0.565 to 113 g/L, more preferably in a range of from 2.825 to 56.5 g/L, and still more preferably in a range of from 5.65 to 28.25 g/L. Note here that the proportion of a whole blood sample added in the reaction solution may satisfy both the above-described percentage by volume and the percentage by weight of Hb or either one of them, for example.

Whole blood may be, for example, any of hemolyzed whole blood, non-hemolyzed whole blood, anticoagulant whole blood, whole blood including coagulation fraction, and the like.

In the present invention, a template nucleic acid contained in a sample is, for example, DNA. The DNA may be, for example, DNA inherently contained in a sample such as a biological sample or the like or amplification product DNA obtained through amplification by nucleic acid amplification. In the latter case, the amplification product DNA may be cDNA produced by a reverse transcription reaction from RNA (total RNA, mRNA, and the like) that is contained inherently in the sample.

In the method for producing an amplification product of the present invention, for example, prior to the initiation of nucleic acid amplification, albumin preferably is further added to the reaction solution. By such addition of albumin, for example, the influence of generated insoluble deposits or turbidity described above can be reduced further, and amplification efficiency also can be enhanced further.

The proportion of albumin added in the reaction solution is, for example, from 0.01% to 2% by weight, preferably from 0.1% to 1% by weight, and more preferably from 0.2% to 0.8% by weight. The albumin is not particularly limited, and examples thereof include bovine serum albumin (BSA), human serum albumin, rat serum albumin, horse serum albumin, and the like. These albumins may be used alone or in a combination of two or more of them.

Next, the method for producing an amplification product of the present invention will be explained with reference to an example where, with respect to a whole blood sample, amplification of a target nucleic acid is conducted using DNA as a template nucleic acid through a PCR using a primer for amplifying the target nucleic acid in the presence of the probe of the present invention. It is to be noted that the present invention is characterized by using the probe of the present invention, and other configurations and conditions are not limited at all.

First, a PCR reaction solution is prepared. Although the proportion of a probe added in the reaction solution is not particularly limited, it is preferable that the probe is added to the reaction solution so that its concentration is in a range of 10 to 400 nmol/L, more preferably in a range of 20 to 200 nmol/L. When a fluorescent substance is used as a label of a probe, a non-labeled probe having the same sequence as that of the labeled probe may be used in combination in order to adjust the fluorescence intensity to be detected, for example. In this non-labeled probe, phosphoric acid may be added to the 3' terminal. In this case, it is preferable that the molar ratio of the labeled probe to the non-labeled probe is, for example, from 1 : 10 to 10 : 1.

Although the proportion of a primer added in the reaction solution is not particularly limited, it is preferable to add the primer so that its concentration is from 0.1 to 2 µmol/L, more preferably from 0.25 to 1.5 µmol/L, and particularly preferably from 0.5 to 1 µmol/L. Further, as the primer, a paired primer set composed of a forward primer and a reverse primer can be used, and in this case, both the primers preferably are added so that their concentrations are in the range described above. The ratio between the forward primer (F) and the reverse primer (R) added (molar ratio F : R) is not particularly limited, and is, for example, preferably from 1 : 0.25 to 1 : 4, more preferably from 1 : 0.5 to 1 : 2. One type or two or more types of primer or primer set may be used, for example. In the present invention, the primer can be used as appropriate depending on the number of target sequences that are desired to be amplified in one reaction solution, for example.

Although the proportion of a whole blood sample in the reaction solution is not particularly limited, it is preferred to be in the range described above. The whole blood sample may be added to the reaction solution as it is or may be added to the reaction solution after being diluted with a solvent such as water, a buffer solution, or the like. When the whole blood sample is previously diluted, the dilution factor is not particularly limited, and for example, it can be set so that the final proportion of the added whole blood in a reaction solution is in the range described above. The dilution factor is, for example, from 100-fold to 2000-fold, and preferably from 200-fold to 10000-fold.

Other composition components in the reaction solution are not particularly limited and may be conventionally known components. The proportions of the components are also not particularly limited. Examples of the composition components include DNA polymerase, nucleotide (nucleoside triphosphate (dNTP)), and a solvent. Further, as described above, it is preferable that the reaction solution further contains albumin. It is to be noted that, in the reaction solution, the order of adding each composition component is not limited at all.

The DNA polymerase is not particularly limited, and for example, conventionally known thermotolerant bacterium-derived polymerase can be used. As specific examples, *Thermus aquaticus*-derived DNA polymerase (USP. Nos. 4,889,818 and 5,079,352) (product name "Taq polymerase"), *Thermus thermophilus-derived* DNA polymerase (WO/1991/009950) (rTth DNA polymerase), *Pyrococcus furiosus-derived* DNA polymerase (WO/1992/009689) (Pfu DNA polymerase: produced by Stratagene), *Thermococcus litoralis-derived* DNA polymerase (EP-A 455 430) (trademark "Vent": produced by New England Biolabs Inc.), *Thermococus kodakaraensis*-derived DNA polymerase (product name "KOD DNA polymerase"), and the like are commercially available. Among them, *Thermus aquaticus*-derived thermotolerant DNA polymerase is preferred.

The proportion of DNA polymerase added in the reaction solution is not particularly limited, and is, for example, from 1 to 100 U/mL, preferably from 5 to 50 U/mL, and more preferably from 20 to 30 U/mL. With regard to the unit of activity (U) of DNA polymerase , 1 U generally is defined as an activity for incorporating 10 nmol of an entire nucleotide into acid-insoluble precipitate per 30 minutes at 74°C in a reaction solution for activity measurement using activated salmon sperm DNA as a template primer. The composition of the reaction solution for activity measurement is, for example, 25 mmol/L TAPS buffer (pH 9.3, 25°C), 50 mmol/L KCL, 2 mmol/L MgCl₂, 1 mmol/L mercaptoethanol, 200 µmol/L dATP, 200 µmol/L dGTP, 200 µmol/L dTTP, 100 µmol/L [α-³²P] dCTP, and 0.25 mg/mL activated salmon sperm DNA.

The nucleotide triphosphate may be generally dNTP (dATP, dCTP, dGTP, dTTP, and/or dUTP). The proportion of dNTP added in the reaction solution is not particularly limited, and is, for example, from 0.01 to 1 mmol/L, preferably from 0.05 to 0.5 mmol/L, and more preferably from 0.1 to 0.3 mmol/L.

Examples of the solvent include buffer solutions such as Tris-HCl, Tricine, MES, MOPS, HEPES, CAPS, and the like, and commercially available buffer solutions for PCR or buffer solutions of commercially available PCR kits can be used.

Further, the PCR solution further may contain glycerol, heparin, betaine, KCl, MgCl₂, MgSO₄, DMSO, and the like, and the proportions of these components added may be set, for example, within a range in which the PCR is not inhibited.

The total volume of the reaction solution is not particularly limited and can be, for example, determined as appropriate depending on a device to be used (thermal cycler), a container such as a tube or a chip etc. However, the total volume is generally from 1 to 500 µL and preferably from 10 to 100 µL.

Next, a PCR is conducted. Generally, a PCR has three steps: (1) disassociation of a double-stranded nucleic acid to single-stranded nucleic acids, (2) annealing of primers to the single-stranded nucleic acids, and (3) elongation of the annealed primers (polymerase reaction), as one cycle. The temperature condition in each step is not particularly limited as long as, as described above, the elongation reaction temperature in the step (3) is higher than the Tm_{Per} value of the probe. As the conditions for conducting the respective steps, the following conditions can be exemplified: for the dissociation step (1), for example, at 90°C to 99°C for 1 to 120 seconds, preferably at 92°C to 95°C for 1 to 60 seconds; for the annealing step (2), for example, at 40°C to 70°C for 1 to 300 seconds, preferably at 50°C to 70°C for 5 to 60 seconds; and for the elongation step (3), for example, at 50°C to 80°C for 5 to 60 seconds, preferably at 50°C to 75°C for 5 to 60 seconds. However the conditions for conducting the respective steps are not limited thereto.

The number of cycles in the PCR is not particularly limited, and preferably is, for example, 30 cycles or more. The upper limit of the number of cycles is not particularly limited, and is, for example, a total of 100 cycles or less, preferably a total of 70 cycles or less, and more preferably a total of 50 cycles or less.

In the above-described manner, a target sequence can be amplified in the presence of the probe of the present invention. According to this method, as described above, since annealing of a primer to a template nucleic acid and elongation from the annealed primer is hardly inhibited by the probe, amplification with higher efficiency than ever before is achieved even in either case where the target sequence contains a perfectly matched complementary strand to the probe or where the target sequence contains a mismatched complementary strand to the probe. The amplification method of the present invention also can be referred to as the method for producing an amplification product of a target sequence.

The amplification method of the present invention further may include the step of: detecting an amplification product of a target nucleic acid obtained by an amplification reaction described above. This allows the presence or absence of the amplification product or a polymorphism in the target sequence to be detected. One aspect of this embodiment will be explained below as a method for analyzing a target sequence of the present invention.

### <Method for analyzing target sequence>

The method for analyzing a target sequence of the present invention is, as described above, a method for analyzing a target nucleic acid sequence using a probe that can hybridize to the target nucleic acid sequence, including the steps of: (A) amplifying the target nucleic acid sequence in a template nucleic acid in the presence of the probe by the amplification method of the present invention; and (B) after the step (A), measuring a signal value showing a molten state of a double-stranded nucleic acid composed of a resultant amplification product and the probe while changing a temperature of a reaction solution in the step (A).

According to the method for analyzing a target sequence of the present invention, for example, the presence or absence of amplification of the target sequence or a polymorphism in the target sequence can be distinguished. It is to be noted that the analysis method of the present invention is characterized by amplifying a target sequence by the amplification method of the present invention, i.e., amplifying a target sequence in the presence of the probe of the present invention, and for example, other configurations or conditions are not limited at all.

Preferably, the method for analyzing a target sequence of the present invention further includes the following step (C), for example. By checking variations in signal value with changes in temperature of the reaction solution as in the step (C), the presence or absence of amplification of the target sequence or a polymorphism can be distinguished as described above. (C) analyzing the target sequence from a variation in the signal value with a change in temperature

In the present invention, the step (C) is preferably a melting profile analysis using a melting profile showing variations in signal value with changes in temperature. A melting profile is generally a graph showing the relationship between each temperature and a signal value showing the molten state of a sample or a differential value of the signal value (hereinafter referred to as "signal differential value"). By using such a melting profile, for example, the presence or absence of a peak at the Tm_{Per} value and the Tm_{Mis} can be determined easily by visual observation, for example.

The melting profile is preferably a graph showing the relationship between the temperatures and the signal differential values because the amount of change in signal is easy to determine. The signal differential value may be represented by, for example, "dF/dT" or "-dF/dT". The dF shows the amount of change in signal value, and the dT shows the amount of change in time. When the generation of signals is suppressed by melting of a sample, a valley-shaped peak is shown in the melting profile representing the signal differential values by "dF/dT", and a mountain-shaped peak is shown in the melting profile representing the signal differential values by "-dF/dT". On the other hand, when signals are generated by melting of a sample, a mountain-shaped peak is shown in the melting profile representing the signal derivative values by "dF/dT", and a valley-shaped peak is shown in the melting profile representing the signal derivative values by "-dF/dT". The signals may be generated by non-melting of the sample and the generation of the signals may be suppressed by melting of the sample, or on the other hand, the generation of the signals may be suppressed by non-melting of the sample and the signals may be generated by melting of the sample, for example. Regardless of whether signals are generated by either melting or non-melting of a sample and whether signal differential values are represented by either of the formulae described above, the magnitude of the peak can be evaluated by the magnitude of the absolute value of the signal differential value.

When a polymorphism of the target sequence is analyzed by the analysis method of the present invention, the target sequence is, for example, a sequence having a target site in which a polymorphism occurs, and the probe of the present invention is a sequence complementary to the sequence. In the step (C), the polymorphism in the target site of the target sequence can be analyzed from variations in the signal value. As described above, in the case where the probe is a mutant-type probe, when a peak is detected at around the Tm_{Per} value (i.e., Tmₘᵤₜ value), the polymorphism can be determined as a mutant type, and when a peak is detected at around the Tm_{Mis} value (i.e., Tm_{wlid} value), the polymorphism can be determined as a wild type. Further, when a peak is detected at either one of the Tm values, the polymorphism can be determined as a homozygous polymorphism, and when a peak is detected at both of the Tm values, the polymorphism is determined as a heterozygous polymorphism. It is to be noted that, in the present invention, polymorphisms in a target site are referred to as a mutant type and a wild type. However, this is merely for the sake of convenience and by no means limits the present invention.

In the step (B), the measurement of signal values showing the molten states of a double-stranded nucleic acid composed of the amplification product and the probe may be measurement of the absorbance at 260nm, or measurement of signals of a labeling substance. Preferably, the former is adopted, for example, when the probe of the present invention is a non-labeled probe. Preferably, the latter is adopted, for example, when the probe of the present invention is a labeled probe labeled with a labeling substance. The labeled probe may be, for example, a labeled probe that shows signals independently and shows no signals when it forms a hybrid, or a labeled probe that shows no signals independently and shows signals when it forms a hybrid. The former probe does not show signals when it forms a hybrid (double-stranded nucleic acid) with a target sequence and shows signals when it is released by heating. On the other hand, the latter probe shows signals when it forms a hybrid (double-stranded nucleic acid) with a target sequence, and the signals are reduced (quenched) when the probe is released by heating. Therefore, by detecting signals by this labeling substance under the condition specific to the signals (absorption wavelength and the like), the determination of the progress of melting of the hybrid and the Tm values, and the like can be achieved as in the case of the measurement of absorbance at 260 nm.

In the present invention, as described above, it is possible to conduct an analysis of two or more target sequences or a polymorphism analysis with respect to two or more target sites in one target sequence in the same reaction solution. In such cases, to a reaction solution of nucleic acid amplification of the step (A), two or more types of the probes of the present invention may be added previously depending on the target sequences or target sites to be analyzed. When two or more types of the probe are used as above, it is preferable that the probes are respectively labeled with different labeling substances that can be detected under different conditions. As above, by using the different labeling substances, analysis of the respective target sequences or the respective target sites can be achieved even in the same reaction solution by changing a detection condition.

Next, the analysis method of the present invention will be explained with reference to an example where a labeled probe is used as the probe of the present invention. However, the present invention is not limited to this example. It is to be noted that the analysis method of the present invention can be conducted in the same manner as the amplification method of the present invention unless otherwise stated.

First, in the same manner as described above, a PCR solution containing a labeled primer and a primer for amplifying a target sequence is prepared, and a PCR is conducted to amplify the target sequence. The reaction solution contains the probe of the present invention, a primer, DNA polymerase, dNTP, a sample containing a template nucleic acid, and the like, for example. Besides these, the reaction solution may contain a variety of additives that can be used for nucleic acid amplification.

Next, disassociation of the obtained amplification product (for example, disassociation from a double-stranded DNA and the like into single-stranded nucleic acids) and hybridization of the disassociated single-stranded nucleic acid and the labeled probe are conducted using the PCR solution as it is. For example, this can be achieved by changing the temperature of the reaction solution.

The heating temperature in the disassociation step is not particularly limited as long as the amplification product can be disassociated at the temperature, and is, for example, from 85°C to 95 °C. The heating time is also not particularly limited, and is, generally, from 1 second to 10 minutes, preferably from 1 second to 5 minutes.

The hybridization of the disassociated single-stranded nucleic acid and the labeled probe can be conducted by, for example, after the disassociation step, lowering the heating temperature in the disassociation step. Although the temperature condition is not particularly limited, when checking whether or not the target sequence is a perfect match to the probe, the temperature is preferably lower than the Tm_{Per} value of the labeled probe. When checking whether or not the target sequence is a mismatch to the probe, and further, when checking whether the target sequence is a perfect match or a mismatch, the temperature is preferably lower than the Tm_{Mis} value. The hybridization temperature is not particularly limited, and is generally from 40°C to 50 °C, for example.

Then, the temperature in the reaction solution is changed, and signal values showing the molten states of the formed hybrid composed of the amplification product and the labeled probe are measured. Specifically, for example, the reaction solution (the formed hybrid composed of the single-stranded DNA and the labeled probe) is heated, and variations in signal value with increase in temperature are measured. As described above, when the probe (guanine quenching probe) in which the terminal base C is labeled is used, fluorescence is reduced (or quenched) in the state where the probe hybridizes with a single-stranded DNA, and fluorescence is generated in the state where the probe is disassociated. Therefore, for example, the formed hybrid with reduced (quenched) fluorescence may be heated gradually, and the increase in fluorescence intensity with increase in temperature may be measured.

A temperature range at the time of measuring variations in fluorescence intensity is not particularly limited. However, for example, the initiation temperature is from room temperature to 85°C, preferably from 25°C to 70°C, and the end temperature is, for example, from 40°C to 105°C. The temperature rising rate is not particularly limited, and is, for example, from 0.1 to 20 °C/sec., preferably from 0.3 to 5 °C/sec.

Next, the target sequence is analyzed based on the variations in the signal. To this end, with respect to the probe of the present invention, a Tm_{Per} value and a Tm_{Mis} value are previously determined. Then, based on the fluorescence intensities obtained, the amount of change in fluorescence intensity per unit time at each temperature is calculated, and it is checked whether or not the maximum absolute value is shown either at the Tm_{Per} value or the Tm_{Mis}. Specifically, for example, a melting profile plotting the temperatures and "-d amount of change in fluorescence intensity/dT" is prepared, and it is checked whether or not a valley-shaped peak showing the minimum value is present either at the Tm_{Per} value or the Tm_{Mis}. For example, when a melting profile plotting the temperatures and "d amount of change in fluorescence intensity/dT" is prepared, it is checked whether or not a mountain-shaped peak showing the maximum value is present either at the Tm_{Per} value or the Tm_{Mis}. As a result of this, if a peak is found at around the Tm_{Per} value, the target sequence can be determined as being a perfect match to the probe, and when a peak is found at around the Tm_{Mis} value, the target sequence can be determined as being a mismatch to the probe. Further, when the probe is a mutant-type detection probe, it can be determined that a polymorphism is a mutant type in the case of perfect match and that a polymorphism is a wild type in the case of mismatch. On the other hand, when the probe is a wild-type detection probe, it can be determined that a polymorphism is a wild type in the case of perfect match and that a polymorphism is a mutant type in the case of mismatch.

Further, when the probe that generates fluorescence in the state of hybridizing with a single-stranded DNA and exhibits reduced (or quenched) fluorescence in the state of being dissociated as opposed to a guanine quenching probe is used, the formed hybrid generating fluorescence may be heated gradually, and the decrease in fluorescence intensity with increase in temperature may be measured.

Further, in the present invention, instead of raising the temperature of a reaction solution containing the probe and measuring variations in signal with the increase in temperature as described above, for example, variations in signal at the time of forming a hybrid may be measured. That is, at the time of forming a hybrid by lowering the temperature of the reaction solution containing the probe, variations in signal with decrease in temperature may be measured.

As a specific example, when a labeled probe (for example, guanine quenching probe) that shows signals independently and shows no signals when it forms a hybrid is used, fluorescence is generated in the state where a single-stranded DNA and the probe are dissociated, but the fluorescence is reduced (or quenched) when a hybrid is formed by lowering the temperature. Therefore, for example, the temperature of the reaction solution may be lowered gradually, and the decrease in fluorescence intensity with decrease in temperature may be measured. On the other hand, when a labeled probe that shows no signals independently and shows signals when it forms a hybrid is used, fluorescence is not generated in the state where a single-stranded DNA and the probe are dissociated, but the fluorescence is generated when a hybrid is formed by lowering the temperature. Therefore, for example, the temperature of the reaction solution may be lowered gradually, and the increase in fluorescence intensity with decrease in temperature may be measured.

### <Method for suppressing amplification inhibition>

The method for suppressing amplification inhibition of the present invention is, as described above, a method for suppressing, in amplification of a target sequence in the presence of a probe, inhibition of the amplification of the target sequence, wherein a method for amplifying the target sequence is the amplification method of the present invention. The present invention is characterized by conducting, in amplification of a target sequence in the presence of the probe of the present invention, the amplification method of the present invention, i.e., amplifying the target sequence in the presence of the probe of the present invention, and other configurations and conditions are not limited at all. It is to be noted that the suppression method of the present invention can be conducted in the same manner as the amplification method of the present invention.

### <Probe>

The probe of the present invention is, as described above, a probe used for the amplification method of the present invention or the method for suppressing amplification inhibition of the present invention. The probe is a probe that forms, with a strand complementary to the probe, a double-stranded nucleic acid whose melting temperature is equal to or lower than a reaction temperature of the elongation reaction. Preferably, the probe forms, with a strand perfectly complementary to the probe, a double-stranded nucleic acid whose melting temperature is equal to or lower than a reaction temperature of the elongation reaction. A specific explanation therefor is as described in connection with the amplification method of the present invention. The probe of the present invention can be used for the suppression method, the amplification method, and the analysis method of the present invention.

### <Amplification reagent>

An amplification reagent of the present invention is a reagent used for the amplification method, the suppression method, or the analysis method of the present invention and contains the probe of the present invention. It is only necessary that the amplification reagent of the present invention contains the probe of the present invention, and other components are not limited at all. The amplification reagent of the present invention may further contain a primer for amplifying a target sequence, polymerase such as DNA polymerase or the like, dNTP, and the like, for example.

The form of the amplification reagent of the present invention is not particularly limited, and the reagent may be, for example, a liquid reagent or a dry reagent to be suspended in a solvent before use. Further, the contents of other components such as the probe of the present invention, the primer, and the like are also not particularly limited. The amplification reagent of the present invention may be an amplification kit, and for example, respective reagents may be contained in the same container or different containers. Furthermore, the amplification kit is preferably provided with instructions.

### <Method for designing probe>

A method for designing a probe of the present invention is a method for designing a probe to be used in a method for amplifying a target nucleic acid sequence in a template nucleic acid in the presence of a probe. The probe is designed so that a melting temperature of a double-stranded nucleic acid composed of the probe and a strand complementary to the probe is equal to or lower than a reaction temperature of the elongation reaction. In the present invention, preferably, the probe is designed so that a melting temperature of a double-stranded nucleic acid composed of the probe and a strand perfectly complementary to the probe is equal to or lower than a reaction temperature of the elongation reaction. Specifically, the design method of the present invention can be conducted in the same manner as the method described in connection with the amplification method of the present invention. The present invention also can be referred to as a probe producing method and the method includes the step of designing a probe in a manner described above.

Next, the present invention will be explained with reference to examples. However, the present invention is not limited to these examples.

### Example 1

From blood of a subject showing a heterozygous CYP2C9*3 polymorphism, a genome was purified using a kit (product name "QIAamp DNA Mini kit", produced by QIAGEN Co., Ltd.). The purified genome was diluted to 1/10 (volume) with TE (10 mmol/L Tris-HCl and 1mmol/L EDTA). 1 µL of this diluted purified genome was added to 49 µL of a PCR reagent of the following composition, and a PCR was conducted using a thermal cycler. The PCR condition was as follows: a treatment at 95°C for 60 seconds, followed by 50 cycles, with a dissociation treatment of a double strand for 1 second and an elongation reaction treatment for 15 seconds as 1 cycle. The temperature of the dissociation treatment was set at 95°C, and the temperature of the elongation reaction treatment (including an annealing treatment) was set at the predetermined temperature (52°C, 58°C, or 60°C). After the PCR, the resultant reaction solution further was treated at 95°C for 1 second and at 40°C for 60 seconds, followed by heating from 40°C to 95°C at a temperature rate of 10°C/3 sec., and the change in fluorescence intensity with time was measured. The measurement wavelength was set at 515 nm to 555 nm (detection of fluorescence dye BODIPY FL).

**[Table 1]**

| (PCR reaction solution: unit µL) | |
|---|---|
| Distilled water | 27.6 |
| 10x Gene Taq buffer (Mg Free)⁽¹⁾ | 5 |
| 10% NaN₃ | 0.2 |
| 5 U/µL Gene Taq FP⁽¹⁾ | 0.25 |
| 80% Glycerol | 9.37 |
| 100 mmol/L MgCl₂ | 0.75 |
| 2.5 mmol/L dNTP | 4 |
| 100 µmol/L CYP2C9 F-primer | 0.5 |
| 100 µmol/L CYP2C9 R-primer | 0.2 |
| 100 µmol/L Probe for CYP2C9*3 | 0.04 |
| 20% BSA | 1 |
| | Total 49 µL |

| | |
|---|---|
| (1) Product name "Gene Taq FP", produced by NIPPON GENE CO., LTD. | |

(Primer set)
   CYP2C9*3 F-primer (SEQ ID NO: 1) 5'-gagcccctgcatgcaa-3'
   CYP2C9*3 R-primer (SEQ ID NO: 2) 5'-gatactatgaatttggggacttcgaa-3'
(Mutant-type detection probe)
   CYP2C9*3 Probe (SEQ ID NO: 3) 5'-gggagaagGtcaAGgTatc-(BODIPY FL)-3'

The Tm_{Per} value of a hybrid (perfectly matched double strand) composed of a mutant-type detection probe for CYP2C9*3 and a strand perfectly complementary to the probe is 58°C. The Tm_{Mis} value of a hybrid (mismatched double strand) composed of the probe for the CYP2C9*3 and a strand perfectly complementary to the probe except for a single base in a target site is 53°C.

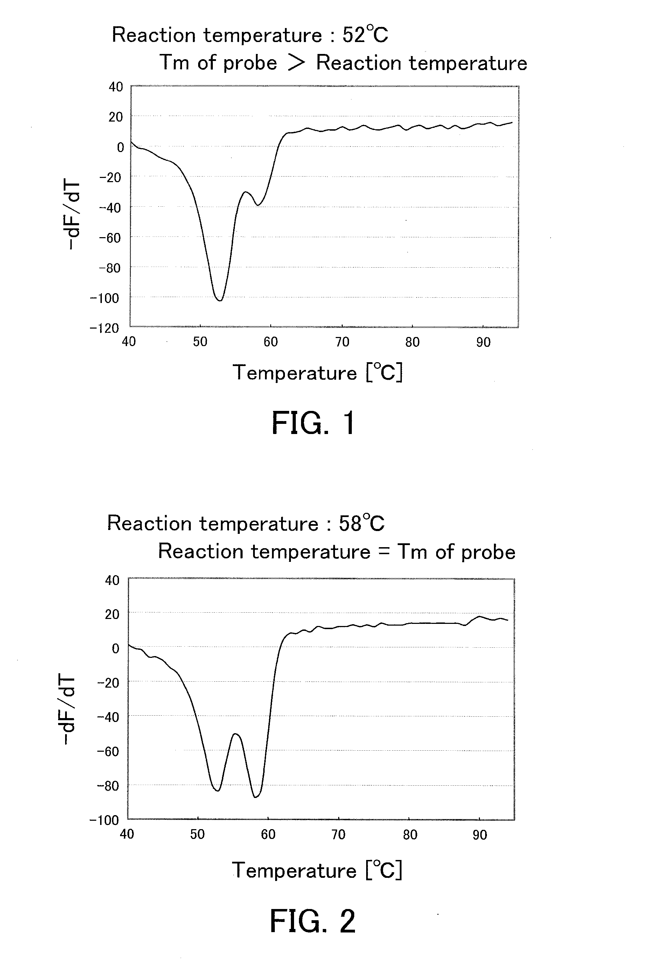

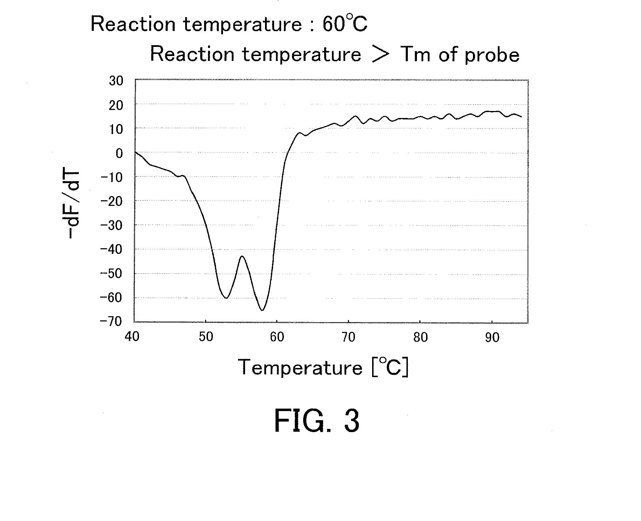

The results are shown in FIGs. 1 to 3. FIGs. 1 to 3 are graphs of melting profiles showing changes in fluorescence intensity with increase in temperature. Differential values on the vertical axis show "-d amount of increase in fluorescence intensity/dT" (-dF/dT), and the horizontal axis shows temperatures. FIG. 1 is a graph showing a melting profile in the case where an elongation reaction temperature of the PCR was set at 52°C, FIG. 2 is a graph showing a melting profile in the case where the elongation reaction temperature was set at 58°C, and FIG. 3 is a graph showing a melting profile in the case where the elongation reaction temperature was set at 60°C.

When the elongation reaction temperature is 52°C, the relationship between the Tm_{Per} value of the perfectly matched double strand and the elongation reaction temperature is "Tm_{Per} value > elongation reaction temperature" and does not satisfy the condition of the present invention. Therefore, as shown in FIG. 1, although the peak of the mismatched double strand at 53°C could be identified well, the peak of a perfectly matched double strand at 58°C was very small, and hardly distinguishable. On the other hand, when the elongation reaction temperature is 58°C, the relationship between the Tm_{Per} value of the perfectly matched double strand and the elongation reaction temperature is "Tm_{Per} value = elongation reaction temperature", and when the elongation reaction temperature is 60°C, the relationship between the Tm value of the perfectly matched double strand and the elongation reaction temperature is "Tm_{Per} value < elongation reaction temperature". In both the cases, the relations satisfy the condition of the present invention. Accordingly, as shown in FIGs. 2 and 3, as well as the peak of perfect match at 58°C, the peak of mismatch at 53°C could be detected.

### Industrial Applicability

As described above, according to the present invention, even when a target sequence is amplified in the presence of a probe, the probe is less prone to hybridize to a template nucleic acid at a reaction temperature of the elongation reaction, whereby annealing of a primer to a target nucleic acid or an elongation reaction from the primer is hardly inhibited by the probe. As a result, it becomes possible to amplify the target sequence sufficiently. Therefore, according to the present invention, for example, in a Tm analysis, the presence or absence of a peak can be distinguished with higher reliability than ever before. Consequently, it can be said that the present invention is very useful technology especially in the field of gene analysis.

### [Sequence listing]

TF08044-01. ST25. txt

## Claims

1. A method for amplifying a target nucleic acid sequence in a template nucleic acid in the presence of a probe, comprising the step of:
amplifying the target nucleic acid sequence by an elongation reaction from a primer in the presence of a probe that can hybridize to the target nucleic acid sequence,
wherein used as the probe is a probe that forms, with a strand complementary to the probe, a double-stranded nucleic acid whose melting temperature is equal to or lower than a reaction temperature of the elongation reaction.

2. The amplification method according to claim 1, wherein used as the probe is a probe that forms, with a strand perfectly complementary to the probe, a double-stranded nucleic acid whose melting temperature is equal to or lower than a reaction temperature of the elongation reaction.

3. The amplification method according to claim 2, wherein a difference between the melting temperature of the double-stranded nucleic acid composed of the probe and the strand perfectly complementary to the probe and the reaction temperature of the elongation reaction is about 0°C or higher.

4. The amplification method according to claim 2, wherein the probe is a probe that forms, with a strand perfectly complementary to the probe except for a single base, a double-stranded nucleic acid whose melting temperature is different from the melting temperature of the double-stranded nucleic acid composed of the probe and the strand perfectly complementary to the probe by about 1°C or higher.

5. The amplification method according to claim 1, wherein the probe is a labeled probe labeled with a labeling substance.

6. The amplification method according to claim 4, wherein the labeling substance is a fluorescent substance.

7. A method for suppressing, in amplification of a target nucleic acid sequence in a template nucleic acid in the presence of a probe, inhibition of the amplification of the target nucleic acid sequence,
wherein a method for amplifying the target nucleic acid sequence is the amplification method according to claim 1.

8. A method for analyzing a target nucleic acid sequence using a probe that can hybridize to the target nucleic acid sequence, comprising the steps of:
(A) amplifying the target nucleic acid sequence in a template nucleic acid in the presence of the probe by the amplification method according to claim 1; and
(B) after the step (A), measuring a signal value showing a molten state of a double-stranded nucleic acid composed of a resultant amplification product and the probe while changing a temperature of a reaction solution in the step (A).

9. The analysis method according to claim 8, further comprising the step of:
(C) analyzing the target sequence from a variation in the signal value with a change in temperature.

10. The analysis method according to claim 9, wherein the step (C) is a melting profile analysis using a melting profile showing the variation in the signal value with the change in temperature.

11. The analysis method according to claim 9, wherein the target nucleic acid sequence comprises a target site to be detected where a polymorphism occurs, and in the step (C), the polymorphism in the target site of the target nucleic acid sequence is analyzed from the variation in the signal value.

12. The analysis method according to claim 9, wherein in the step (C), the presence or absence of amplification of the target nucleic acid sequence is analyzed from the variation in the signal value.

13. The analysis method according to claim 8, wherein the probe is a labeled probe labeled with a labeling substance.

14. The analysis method according to claim 13, wherein the labeling substance is a fluorescent substance, and the signal value in the step (B) is a fluorescence intensity.

15. A probe to be used for the amplification method according to claim 1, wherein the probe is a probe that forms, with a strand complementary to the probe, a double-stranded nucleic acid whose melting temperature is equal to or lower than a reaction temperature of the elongation reaction.

16. The probe according to claim 15, wherein the probe is a probe that forms, with a strand perfectly complementary to the probe, a double-stranded nucleic acid whose melting temperature is equal to or lower than the reaction temperature of the elongation reaction.

17. The probe according to claim 16, wherein a difference between the melting temperature of the double-stranded nucleic acid composed of the probe and the strand perfectly complementary to the probe and the reaction temperature of the elongation reaction is about 0°C or higher.

18. The probe according to claim 16, wherein a difference between the melting temperature of the double-stranded nucleic acid composed of the probe and the strand perfectly complementary to the probe and a melting temperature of a double-stranded nucleic acid composed of the probe and a strand perfectly complementary to the probe except for a single base is about 1°C or higher.

19. The probe according to claim 15, wherein the probe is a labeled probe labeled with a labeling substance.

20. The probe according to claim 19, wherein the labeling substance is a fluorescent substance.

21. A method for designing a probe to be used for a method for amplifying a target nucleic acid sequence in a template nucleic acid in the presence of a probe,
wherein the probe is designed so that a melting temperature of a double-stranded nucleic acid composed of the probe and a strand complementary to the probe is equal to or lower than a reaction temperature of an elongation reaction.

22. The probe design method according to claim 21, wherein the probe is designed so that a melting temperature of a double-stranded nucleic acid composed of the probe and a strand perfectly complementary to the probe is equal to or lower than the reaction temperature of the elongation reaction.


SEQUENCE LISTING

<110>  ARKRAY, Inc.

<120>  Methods for amplification of target nucleic acid seaquence and probe
using thereof

<130>  P27605-WO-EP

<140>  08865229.2
<141>  2008-12-25

<150>  JP2007/334360
<151>  2007-12-26

<160>  3

<170>  PatentIn version 3.1

<210>  1
<211>  16
<212>  DNA
<213>  Artificial

<220>
<223>  forward primer

<400>  1
gagcccctgc atgcaa                                                    16


<210>  2
<211>  26
<212>  DNA
<213>  Artificial

<220>
<223>  reverse primer

<400>  2
gatactatga atttggggac ttcgaa                                         26


<210>  3
<211>  19
<212>  DNA
<213>  Artificial

<220>
<223>  probe

<400>  3
gggagaaggt caaggtatc                                                 19

SEQUENCE LISTING

<110>  ARKRAY, Inc.

<120>  Methods for amplification of target nucleic acid seaquence and probe
using thereof

<130>  TF08044-01

<150>  JP2007/334360
<151>  2007-12-26

<160>  3

<170>  PatentIn version 3.1

<210>  1
<211>  16
<212>  DNA
<213>  Artificial

<220>
<223>  forward primer

<400>  1
gagcccctgc atgcaa                                                    16


<210>  2
<211>  26
<212>  DNA
<213>  Artificial

<220>
<223>  reverse primer

<400>  2
gatactatga atttggggac ttcgaa                                         26


<210>  3
<211>  19
<212>  DNA
<213>  Artificial

<220>
<223>  probe

<400>  3
gggagaaggt caaggtatc                                                 19